# EUROPÄISCHE PATENTSCHRIFT

(11) **EP 2 177 240 B1**
(45) Veröffentlichungstag und Bekanntmachung des Hinweises auf die Patenterteilung: **07.12.2011**
(21) Anmeldenummer: 08166894.9
(22) Anmeldetag: 17.10.2008
(51) Int. Cl.: A61M 11/06, A61M 16/00

(54) **Kompressor-Vernebler-Gerät für die Inhalationstherapie**
Compressor-atomiser device for inhalation therapy
Appareil compresseur-nébuliseur pour la inhalothérapie

(43) Veröffentlichungstag der Anmeldung: 21.04.2010
(73) Patentinhaber: Pari GmbH, 82319 Starnberg (DE)
(72) Erfinder: Rosenbeiger, Sven, 82319, Starnberg (DE); Kreutzmann, Vera, 82064, Strasslach DIngharting (DE); Deuschl, Michael, 83646, Bad Tölz (DE); Zemke, Robert, 80339 München (DE)
(74) Vertreter: HOFFMANN EITLE

(56) Entgegenhaltungen:
- WO-A-2005/013879
- DE-A1- 10 022 795
- US-A- 4 949 715
- US-A- 5 022 587

## Beschreibung

### Technisches Gebiet

Die Erfindung betrifft ein Kompressor-Vernebler-Gerät für die Inhalationstherapie, mit einem Vernebler mit einer Verneblerdüse für die Vernebelung eines Mediums mit Hilfe von Druckluft und mit einem Kompressor für die Bereitstellung der der Verneblerdüse zuzuführenden Druckluft.

Solche Kompressor-Vernebler-Geräte werden insbesondere zur Vernebelung von medikamenthaltigen Flüssigkeiten für Aerosoltherapieanwendungen verwendet, um Patienten mit Erkrankungen der Atemwege geeignete Medikamente in Form eines Aerosols zu verabreichen. Dabei atmet der Patient das vernebelte Medikament durch den Mund über ein Mundstück ein.

Verneblerdüsen, die mit Hilfe von zugeführter Druckluft aus einer Flüssigkeit ein Aerosol erzeugen, haben sich für die Erzeugung des Aerosols zur Durchführung einer solchen Inhalationstherapie neben anderen Primäraerosolerzeugern in besonderem Maße bewährt.

Der Kompressor, der die Druckluft für die Aerosolerzeugung bereitstellt, weist in der Regel einen Elektromotor als Antrieb auf.

### Stand der Technik

Kompressor-Vernebler-Geräte der eingangs genannten Art sind an sich bekannt, beispielsweise aus dem Deutschen Patent DE 10 2005 022 339.7.

Bei solchen Geräten wird meist ein Kompressor zusammen mit einem austauschbaren Vernebler verwendet. So können unterschiedliche Vernebler mit ein und demselben Kompressor verwendet werden, beispielsweise auch solche, die speziell an die Bedürfnisse von Kindern angepasst sind. Abgesehen davon ist es oft auch wünschenswert, den Vernebler nach längerem Gebrauch durch einen neuen zu ersetzen.

Grundsätzlich ist es dabei wünschenswert, dass sich die Leistung des Kompressor-Vernebler-Geräts und insbesondere der Druck, unter dem das vernebelte Medikament ausgegeben wird, durch den Austausch des Verneblers nicht ändert.

Die Durchmesser der einzelnen Verneblerdüsen weichen jedoch produktionsbedingt leicht von einem Soll-Durchmesser ab. Dadurch variiert bei der Verwendung von verschiedenen Verneblerdüsen mit ein und demselben Kompressoren der Düsendruck. Ein größerer Düsendurchmesser führt dabei zu einem geringeren Düsendruck. Ein reduzierter Düsendruck geht seinerseits mit einem geringeren Luftstrom durch die Düse einher. Dadurch kann weniger Aerosol erzeugt werden. Die Menge an vernebelter Flüssigkeit pro Zeiteinheit, die als "Total Output Rate (TOR)" bezeichnet wird, verringert sich.

Durch diese Varianz des Aerosolspektrums ändert sich die Lungengängigkeit des vernebelten Mediums und damit unter Umständen auch der Therapieerfolg, was insbesondere bei Geräten für medizinische Studien unerwünscht ist.

US-A-5 022 587 offenbart ein Kompressor-Vernebler-Gerät für die Inhalationstherapie gemäß dem Oberbegriff des Patentanspruchs 1 sowie ein Verfahren gemäß dem Oberbegriff des Patentanspruchs 5.

Aus US-A-4 949 715 ist ein vergleichbares Gerät bekannt.

### Darstellung der Erfindung

Der vorliegenden Erfindung liegt die Aufgabe zugrunde, ein Kompressor-Vernebler-Gerät für die Inhalationstherapie zu schaffen, mittels dessen auch bei der Verwendung von unterschiedlichen Verneblerdüsen stets ein konstanter Düsendruck erzeugt wird, sowie ein zugehöriges Verfahren zur Regelung eines Kompressor-Vernebler-Geräts.

Diese Aufgabe wird durch ein Gerät gemäß dem Patentanspruch 1 sowie ein Verfahren gemäß dem Patentanspruch 5 gelöst.

Demzufolge wird der Druck der an der Verneblerdüse bereitgestellten Druckluft bestimmt. Mittels eines integrierten Schaltkreises wird der erfasste Düsendruck mit einem zuvor in dem integrierten Schaltkreis hinterlegten Sollwert verglichen und die Versorgungsspannung des Kompressormotors variiert, bis der erfasste Düsendruck dem Sollwert entspricht.

Während des Betriebs des Geräts wird somit der Düsendruck bestimmt, und Abweichungen vom Sollwert werden durch die Regelung der Versorgungsspannung des Kompressormotors korrigiert. Der integrierte Schaltkreis regelt die Versorgungsspannung des Motors so, dass der Düsendruck auch bei Verneblern mit von einem Normwert abweichenden Düsendurchmessern weitgehend gleich bleibt.

Bevorzugte optionale Merkmale sind in den abhängigen Patentansprüchen beschrieben.

Der Druck der an der Verneblerdüse bereitgestellten Druckluft kann direkt erfasst werden, beispielsweise mittels eines im Kompressor vorgesehenen Drucksensors.

Alternativ oder zusätzlich kann der Düsendruck aus der Stromaufnahme und der Drehzahl des Kompressormotors ermittelt werden. Mit Hilfe dieser beiden Parameter kann der aktuelle Arbeitspunkt des Geräts eindeutig bestimmt werden, und daraus können Rückschlüsse auf den aktuellen Düsendruck gezogen werden. Die dazu notwendigen Kurven werden in Vorversuchen für den jeweiligen Kompressortypen ermittelt.

In dieser Variante werden die Vorgabewerte für Drehzahl und Stromaufnahme des Kompressormotors, beispielsweise im Rahmen einer Qualitätsprüfung anschließend an die Produktion, unter Verwendung einer genormten Düse anhand des Düsendrucks ermittelt. Diese Werte werden dauerhaft im integrierten Schaltkreis hinterlegt.

Im Rahmen dieser Einstellung der Vorgabewerte kann auch die Programmierung anderer Funktionen des integrierten Schaltkreises erfolgen.

Zur Erfassung der Stromaufnahme des Kompressormotors kann beispielsweise ein niederohmiger Shunt-Widerstand Verwendung finden, zur Erfassung der Drehzahl des Kompressormotors beispielsweise ein Hall-Sensor. Es ist jedoch auch möglich, die Drehzahl des Kompressormotors durch das Erfassen von drehzahlabhängigen Schwankungen in der Stromaufnahme zu bestimmen.

In jedem Fall kann der Düsendruck während des Betriebs des Geräts, direkt und/oder durch die Auswertung von Stromaufnahme und Drehzahl, kontinuierlich geregelt werden. Alternativ ist es auch denkbar, den Düsendruck nur in bestimmten Abständen oder auch nur einmal nach dem Einschalten des Geräts auf den Sollwert einzuregeln.

Schließlich kann es von Vorteil sein, in dem integrierten Schaltkreis außerdem einen Anfangswert für die Versorgungsspannung des Kompressormotors zu hinterlegen.

Der Hintergrund dieser Maßnahme ist, dass herstellungsbedingt nicht nur die Verneblerdüsen, sondern auch alle übrigen Einzelteile des Kompressor-Vernebler-Geräts und insbesondere des Kompressors leichte Abweichungen von den Sollmaßen und Schwankungen in den Materialeigenschaften aufweisen können. Aus diesen Abweichungen und Schwankungen resultieren ebenfalls Schwankungen im Düsendruck.

Um auch diese Schwankungen zu vermeiden, wird für jeden einzelnen Kompressor - wiederum beispielsweise im Rahmen einer sich an die Produktion anschließenden Qualitätsprüfung - diejenige Versorgungsspannung ermittelt, bei der dieser Kompressor unter Verwendung einer Normdüse einen gewünschten Düsendruck liefert. Dadurch können auch Schwankungen des Düsendrucks vermieden werden, die aus Bauteiltoleranzen und unterschiedlichen Motorleistungen resultieren.

Dieser Spannungswert wird anschließend in dem integrierten Schaltkreis hinterlegt und bildet einen Anfangswert für die erfindungsgemäße, düsendruckabhängige Regelung der Versorgungsspannung, die dann zusätzlich die durch die unterschiedlichen Düsendurchmesser verursachten Düsendruckschwankungen ausgleicht.

Ausführliche Beschreibung einer bevorzugten Ausführungsform der Erfindung

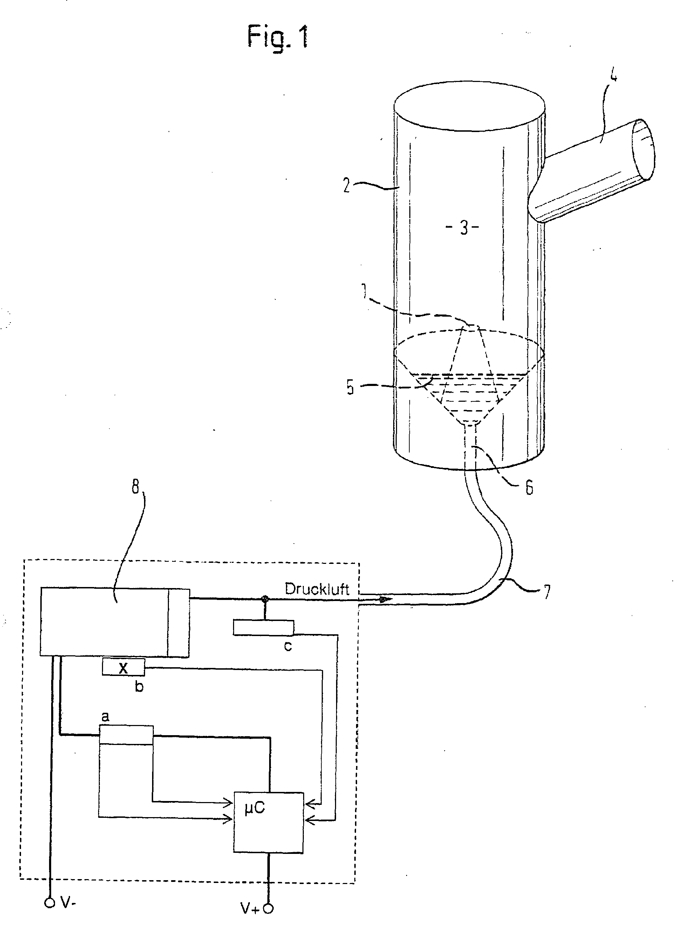

Die anliegende Figur 1 zeigt ein Ausführungsbeispiel eines erfindungsgemäßen Kompressor-Vernebler-Geräts für die Inhalationstherapie.

Das Kompressor-Vernebler-Gerät umfasst eine Verneblerdüse 1, die in einem Verneblergehäuse 2 angeordnet ist, das einen Verneblungsraum 3 umschließt. Über ein Mundstück 4 kann ein Patient ein von der Veneblerdüse 1 erzeugtes Aerosol aus dem Verneblungsraum 3 einatmen. Für die Erzeugung des Aerosols werden der Verneblerdüse 1 eine Flüssigkeit 5, die vorteilhafterweise in dem Verneblergehäuse 2 bevorratet wird, und Druckluft 6 zugeführt.

Die Druckluft 6, die vorzugsweise über eine Schlauchleitung 7 oder ähnliches zu der Verneblerdüse 1 geleitet wird, wird von einem Kompressor bereitgestellt. Dazu umfasst der Kompressor typischerweise eine Drucklufterzeugungseinrichtung (nicht dargestellt), beispielsweise eine Kolben-, Membran- oder Zentrifugalpumpe, und eine elektromotorische Antriebseinrichtung, hier einen Elektromotor 8.

Auf einer Hauptplatine des Kompressors befindet sich neben der Sicherheitselektronik und anderer Schaltungen (Ladeelektronik, Anzeigeelemente, etc.) auch ein programmierbarer integrierter Schaltkreis (in der Zeichnung mit µC gekennzeichnet). Dieser ist in der Lage, vorher definierte Geräteparameter anhand vorgegebener Werte zu überwachen und zu regeln.

Während des Betriebs des Kompressor-Vernebler-Geräts wird nun der Druck der an der Verneblerdüse 1 bereitgestellten Druckluft erfasst. In der vorliegenden Ausführungsform ist hierzu ein Drucksensor c im Kompressor vorgesehen. Mittels des integrierten Schaltkreises µC wird der erfasste Düsendruck mit einem zuvor in dem integrierten Schaltkreis µC hinterlegten Sollwert verglichen und die Versorgungsspannung des Kompressormotors 8 variiert, bis der erfasste Düsendruck dem Sollwert entspricht.

In Fig. 1 ist darüber hinaus ein niederohmiger Shunt-Widerstand a zur Erfassung der Stromaufnahme des Kompressormotors 8 vorgesehen sowie ein Hall-Sensor b zur Erfassung der Drehzahl des Kompressormotors 8, so dass der Düsendruck auch auf der Grundlage der Stromaufnahme und Drehzahl bestimmt werden kann. Mit Hilfe dieser beiden Parameter kann der aktuelle Arbeitspunkt des Geräts eindeutig bestimmt werden, und daraus können Rückschlüsse auf den aktuellen Düsendruck gezogen werden. Die dazu notwendigen Kurven werden in Vorversuchen für den jeweiligen Kompressortypen ermittelt.

In dieser Alternative werden mittels des integrierten Schaltkreises µC die erfasste Stromaufnahme und Drehzahl des Kompressormotors 8 mit zuvor in dem integrierten Schaltkreis µC hinterlegten Sollwerten verglichen und die Versorgungsspannung des Kompressormotors 8 variiert, bis die erfasste Stromaufnahme und Drehzahl den Sollwerten entsprechen.

In der Praxis reicht es natürlich aus, den Düsendruck entweder - beispielsweise mittels des Sensors c - direkt zu erfassen oder wie gerade beschrieben aus der Stromaufnahme und Drehzahl zu bestimmen.

In jedem Fall sorgt der integrierte Schaltkreis µC so für eine Anpassung der Versorgungsspannung des Kompressormotors 8, so dass stets der gewünschte Düsendruck erzeugt wird.

Die Regelung der Versorgungsspannung des Kompressormotors 8 kann über den integrierten Schaltkreis µC selbst erfolgen, wenn dieser so gewählt ist, dass er grundsätzlich mit dieser Funktion ausgestattet ist und außerdem die notwendige Stromstärke den für ihn zulässigen Wert nicht übersteigt. Alternativ kann diese Regelung aber auch über ein separates Bauteil erfolgen.

In der vorliegenden Ausführungsform sind der Kompressor mit seinem Motor 8, der integrierte Schaltkreis µC, der Drucksensor c, der Shunt-Widerstand a sowie der Hall-Sensor b in einem Gehäuse untergebracht, das in Fig. 1 durch eine gestrichelte Linie angedeutet ist.

Im Allgemeinen wird es nicht notwendig sein, die im integrierten Schaltkreis µC hinterlegten Informationen nach einmal abgeschlossener Programmierung zu verändern. Der Benutzer des Kompressor-Vernebler-Geräts, d.h. der Patient, sollte daher zweckmäßigerweise am Zugriff auf den integrierten Schaltkreis gehindert werden. Dies kann beispielsweise dadurch erfolgen, dass der Kompressor mit dem integrierten Schaltkreis und den übrigen Bauteilen erst nach der Programmierung in das Gehäuse eingebracht wird, so dass ein zugehöriger Programmieranschluss geeignet in dem Gehäuse verborgen werden kann.

## Patentansprüche

1. Kompressor-Vernebler-Gerät für die Inhalationstherapie, mit einem Vernebler mit einer Verneblerdüse (1) für die Vernebelung eines Mediums (5) mit Hilfe von Druckluft (6) und mit einem Kompressor für die Bereitstellung der der Verneblerdüse (1) zuzuführenden Druckluft (6), wobei
der Kompressor mittels eines Kompressormotors (8) angetrieben wird und über einen integrierten Schaltkreis (µC) zur Steuerung der Versorgungsspannung des Kompressormotors (8) verfügt, und
das Kompressor-Vernebler-Gerät außerdem Mittel zum Bestimmen des Drucks der an der Verneblerdüse (1) bereitgestellten Druckluft (6) umfasst,
**dadurch gekennzeichnet, dass**
der integrierte Schaltkreis (µC) dazu ausgestaltet ist, den erfassten Düsendruck mit einem zuvor in dem integrierten Schaltkreis (µC) hinterlegten Sollwert zu vergleichen und die Versorgungsspannung des Kompressormotors (8) zu variieren, bis der erfasste Düsendruck dem Sollwert entspricht.

2. Kompressor-Vernebler-Gerät nach Patentanspruch 1, bei welchem Mittel zur Erfassung des Drucks der an der Verneblerdüse (1) bereitgestellten Druckluft (6) vorgesehen sind, vorzugsweise in Form eines Drucksensors (c) im Kompressor.

3. Kompressor-Vernebler-Gerät nach Patentanspruch 1 oder 2, weiter mit Mitteln zum Erfassen der Stromaufnahme des Kompressormotors (8), vorzugsweise einem niederohmigen Shunt-Widerstand (a).

4. Kompressor-Vernebler-Gerät nach einem der vorangehenden Patentansprüche, weiter mit Mitteln zum Erfassen oder anderweitigen Bestimmen der Drehzahl des Kompressormotors (8), vorzugsweise einem Hall-Sensor (b).

5. Verfahren zur Regelung eines Kompressor-Vernebler-Geräts für die Inhalationstherapie, mit einem Vernebler mit einer Verneblerdüse (1) für die Vernebelung eines Mediums (5) mit Hilfe von Druckluft (6) und mit einem Kompressor für die Bereitstellung der der Verneblerdüse (1) zuzuführenden Druckluft (6), wobei
der Kompressor mittels eines Kompressormotors (8) angetrieben wird und über einen integrierten Schaltkreis (µC) zur Steuerung der Versorgungsspannung des Kompressormotors (8) verfügt, und
der Druck der an der Verneblerdüse (1) bereitgestellten Druckluft (6) bestimmt wird,
**dadurch gekennzeichnet, dass**
mittels des integrierten Schaltkreises (µC) der erfasste Düsendruck mit einem zuvor in dem integrierten Schaltkreis (µC) hinterlegten Sollwert verglichen wird und die Versorgungsspannung des Kompressormotors (8) variiert wird, bis der erfasste Düsendruck dem Sollwert entspricht.

6. Verfahren nach Patentanspruch 5, bei welchem der Druck der an der Verneblerdüse (1) bereitgestellten Druckluft erfasst wird, vorzugsweise mittels eines im Kompressor vorgesehenen Drucksensors (c).

7. Verfahren nach Patentanspruch 5 oder 6, bei welchem die Stromaufnahme des Kompressormotors (8) erfasst wird, vorzugsweise mittels eines niederohmigen Shunt-Widerstands (a).

8. Verfahren nach einem der Patentansprüche 5 bis 7, bei welchem die Drehzahl des Kompressormotors (8), vorzugsweise mittels eines Hall-Sensors (b), erfasst oder anderweitig bestimmt wird.

9. Verfahren nach den Patentansprüchen 7 und 8, bei welchem die Drehzahl des Kompressormotors (8) durch das Erfassen von drehzahlabhängigen Schwankungen in der Stromaufnahme bestimmt wird.

10. Verfahren nach einem der Patentansprüche 5 bis 9, bei welchem der Druck der an der Verneblerdüse (1) bereitgestellten Druckluft während des Betriebs des Geräts kontinuierlich geregelt wird.

11. Verfahren nach einem der Patentansprüche 5 bis 10, bei welchem in dem integrierten Schaltkreis (µC) außerdem ein Anfangswert für die Versorgungsspannung des Kompressormotors (8) hinterlegt ist.

## Claims

1. Compressor-nebuliser device for inhalation therapy, having a nebuliser with a nebuliser nozzle (1) for nebulising a medium (5) with the aid of compressed air (6) and having a compressor for providing the compressed air (6) to be supplied to the nebuliser nozzle (1), wherein the compressor is driven by means of a compressor motor (8) and has an integrated circuit (µC) to control the supply voltage of the compressor motor (8), and the compressor-nebuliser device additionally comprises means for determining the pressure of the compressed air (6) provided at the nebuliser nozzle (1), **characterised in that** the integrated circuit (µC) is designed to compare the recorded nozzle pressure with a theoretical value previously registered in the integrated circuit (µC) and to vary the supply voltage of the compressor motor (8) until the recorded nozzle pressure corresponds to the theoretical value.

2. Compressor-nebuliser device according to patent claim 1, in which means are provided to record the pressure of the compressed air (6) provided at the nebuliser nozzle (1), preferably in the form of a pressure sensor (c) in the compressor.

3. Compressor-nebuliser device according to patent claim 1 or 2, further having means for recording the current consumption of the compressor motor (8), preferably a low-impedance shunt resistance (a).

4. Compressor-nebuliser device according to one of the preceding patent claims, further having means for recording or otherwise determining the speed of the compressor motor (8), preferably a Hall sensor (b).

5. Method for regulating a compressor-nebuliser device for inhalation therapy, having a nebuliser with a nebuliser nozzle (1) for nebulising a medium (5) with the aid of compressed air (6) and having a compressor for providing the compressed air (6) to be supplied to the nebuliser nozzle (1), wherein the compressor is driven by means of a compressor motor (8) and has an integrated circuit (µC) to control the supply voltage of the compressor motor (8), and the pressure or the compressed air (6) provided at the nebuliser nozzle (1) is determined, **characterised in that** by means of the integrated circuit (µC), the recorded nozzle pressure is compared with a theoretical value previously registered in the integrated circuit (µC) and the supply voltage of the compressor motor (8) is varied until the recorded nozzle pressure corresponds to the theoretical value.

6. Method according to patent claim 5, in which the pressure of the compressed air provided at the nebuliser nozzle (1) is recorded, preferably by means of a pressure sensor (c) provided in the compressor.

7. Method according to patent claim 5 or 6, in which the current consumption of the compressor motor (8) is recorded, preferably by means of a low-impedance shunt resistance (a).

8. Method according to one of patent claims 5 to 7, in which the speed of the compressor motor (8) is recorded or otherwise determined, preferably by means of a Hall sensor (b).

9. Method according to patent claims 7 and 8, in which the speed of the compressor motor (8) is determined by recording speed-dependent variations in the current consumption.

10. Method according to one of patent claims 5 to 9, in which the pressure of the compressed air provided at the nebuliser nozzle (1) is regulated continuously during operation of the device.

11. Method according to one of patent claims 5 to 10, in which additionally an initial value for the supply voltage of the compressor motor (8) is registered in the integrated circuit (µC).

## Revendications

1. Appareil nébuliseur à compresseur pour l'inhalothérapie, comportant un nébuliseur avec une buse de nébuliseur (1) pour nébuliser un fluide (5) à l'aide d'air comprimé (6) et comportant un compresseur pour fournir l'air comprimé (6) à envoyer à la buse de nébuliseur (1),
le compresseur étant entraîné au moyen d'un moteur de compresseur (8) et disposant d'un circuit intégré (µC) pour la commande de la tension d'alimentation du moteur de compresseur (8)
et le nébuliseur à compresseur comprenant en outre des moyens pour déterminer la pression de l'air comprimé (6) fourni à la buse de nébuliseur (1),
**caractérisé en ce que** le circuit intégré (µC) est conçu pour comparer la pression de buse détectée à une valeur de consigne enregistrée préalablement dans le circuit intégré (µC) et pour modifier la tension d'alimentation du moteur de compresseur (8) jusqu'à ce que la pression de buse détectée corresponde à la valeur de consigne.

2. Appareil nébuliseur à compresseur selon la revendication 1, dans lequel il est prévu des moyens pour détecter la pression de l'air comprimé (6) fourni à la buse de nébuliseur (1), de préférence sous la forme d'un capteur de pression (c) dans le compresseur.

3. Appareil nébuliseur à compresseur selon la revendication 1 ou 2, comportant aussi des moyens pour détecter la consommation de courant du moteur de compresseur (8), de préférence une résistance shunt de faible valeur ohmique (a).

4. Appareil nébuliseur à compresseur selon l'une des revendications précédentes, comportant aussi des moyens pour détecter ou déterminer d'une autre manière la vitesse de rotation du moteur de compresseur (8), de préférence un capteur à effet Hall (b).

5. Procédé de régulation d'un appareil nébuliseur à compresseur pour l'inhalothérapie comportant un nébuliseur avec une buse de nébuliseur (1) pour nébuliser un fluide (5) à l'aide d'air comprimé (6) et comportant un compresseur pour fournir l'air comprimé (6) à envoyer à la buse de nébuliseur (1),
le compresseur étant entraîné au moyen d'un moteur de compresseur (8) et disposant d'un circuit intégré (µC) pour la commande de la tension d'alimentation du moteur de compresseur (8) et la pression de l'air comprimé (6) fourni à la buse de nébuliseur (1) étant déterminée,
**caractérisé en ce que**, au moyen du circuit intégré (µC), on compare la pression de buse détectée à une valeur de consigne enregistrée préalablement dans le circuit intégré (µC) et on modifie la tension d'alimentation du moteur de compresseur (8) jusqu'à ce que la pression de buse détectée corresponde à la valeur de consigne.

6. Procédé selon la revendication 5, selon lequel on détecte la pression de l'air comprimé fourni à la buse de nébuliseur (1), de préférence au moyen d'un capteur de pression (c) prévu dans le compresseur.

7. Procédé selon la revendication 5 ou 6, selon lequel on détecte la consommation de courant du moteur de compresseur (8), de préférence au moyen d'une résistance shunt de faible valeur ohmique (a).

8. Procédé selon l'une des revendications 5 à 7, selon lequel on détecte ou détermine d'une autre manière la vitesse de rotation du moteur de compresseur (8), de préférence au moyen d'un capteur à effet Hall (b).

9. Procédé selon les revendications 7 et 8, selon lequel on détermine la vitesse de rotation du moteur de compresseur (8) en détectant des variations, dépendantes de la vitesse de rotation, dans la consommation de courant.

10. Procédé selon l'une des revendications 5 à 9, selon lequel, pendant le fonctionnement de l'appareil, on régule de manière continue la pression de l'air comprimé fourni à la buse de nébuliseur (1).

11. Procédé selon l'une des revendications 5 à 10, selon lequel est enregistrée en outre dans le circuit intégré (µC) une valeur initiale pour la tension d'alimentation du moteur de compresseur (8).
